# EUROPÄISCHE PATENTSCHRIFT

(11) **EP 2 542 162 B1**
(45) Veröffentlichungstag und Bekanntmachung des Hinweises auf die Patenterteilung: **26.05.2021**
(21) Anmeldenummer: 11711444.7
(22) Anmeldetag: 01.03.2011
(51) Int. Cl.: A61B 17/22, A61B 17/3207

(54) **FÜHRUNGSDRAHT-/KATHETERGEFÜHRTES VALVULOTOM**
VALVULOTOME GUIDED BY A GUIDE WIRE/CATHETER
VALVULOTOME GUIDÉ PAR UN CÂBLE DE GUIDAGE OU UN CATHÉTER

(30) Priorität: 01.03.2010 DE 102010009723
(43) Veröffentlichungstag der Anmeldung: 09.01.2013
(73) Patentinhaber: Andramed GmbH, 72766 Reutlingen (DE)
(72) Erfinder: KOHL, Gunter, 56068 Koblenz (DE); SCHADE, Heinz, 72766 Reutlingen (DE); TENHOLT, Matthias, 68165 Mannheim (DE); KENNEL, Stefan, 68165 Mannheim (DE)
(74) Vertreter: Thiel, Christian
(86) Internationale Anmeldenummer: PCT/EP2011/000981
(87) Internationale Veröffentlichungsnummer: WO 2011/107249

(56) Entgegenhaltungen:
- US-A- 5 158 564
- US-A- 5 178 625
- US-A- 5 282 484
- US-A1- 2005 125 016
- US-A1- 2005 192 606
- US-A1- 2008 255 595

## Beschreibung

Die Erfindung betrifft ein führungsdraht-/kathetergeführtes Valvulotom, mit dem aus einer zur Transplantation bestimmten Körpervene Venenklappen entfernt werden können.

Bei der Transplantation von körpereigenen Venen in den Herzkranzbereich zur Erstellung von Bypässen ist es erforderlich, die dafür bestimmten Venen zuvor von Venenklappen zu befreien. Venenklappen sind geeignet, den Blutfluss zu behindern, was bei Herzkranzgefäßen, die der Versorgung des Herzmuskels mit arteriellem Blut dienen, unerwünscht ist. Insofern müssen für Bypässe bestimmte körpereigene Venen, die zumeist aus dem Oberschenkelbereich des Patienten entnommen werden, zuvor präpariert werden. Eine solche Präparation findet in der Regel, wenn genügend Zeit bleibt, einige Zeit vor der Bypass-Operation im Körper des Patienten statt, so dass die entsprechenden Gefäße Zeit haben, sich zu erholen und zu verheilen.

Zur Entfernung von Venenklappen im Körper eines Patienten werden sog. Valvulotome eingesetzt, d.h. endovaskuläre Instrumente, die in das entsprechende Gefäß eingeführt werden und mit Hilfe von seitlich angeordneten Schneidmessern die Venenklappen abscheren. In der Regel erfolgt ein solcher Eingriff mit Hilfe eines Katheters, der am distalen Unterschenkel über die Leiste oder einen anderen Zugangsort in das entsprechende Gefäß des Patienten geführt wird, und durch den anschließend ein an und für sich bekanntes Valvulotom an den Einsatzort transferiert wird. Nach Verlassen des Katheters entfernen die Schneidelemente des Valvulotoms die Venenklappen, die über ein korbähnliches Gebilde in der Nähe der Valvulotomspitze in den Katheter gezogen und mit dem Katheter und dem Valvulotom aus dem Körper des Patienten entfernt werden.

Ein derartiges Valvulotom ist beispielsweise aus der WO 96/33662 A1 bekannt, das über einen Draht geführt wird, über einen Korb verfügt sowie über Schneidwerkzeuge, die sich vor dem Korb befinden. Distal befindet sich eine atraumatische Spitze.

Dieses Valvulotom wird durch einen Katheter an den Einsatzort geführt, ist aber nicht selbst steuerbar. Insbesondere ist dieses Valvulotom nicht mit einem Führungsdraht, der Steuerungszwecken dient, kombinierbar.

Des Weiteren sind in diesem bekannten Valvulotom Korb- und Schneidelemente voneinander getrennt, müssen aber zusammenwirken. Die Schneidelemente sind beweglich in einem Schneidkopf angeordnet und werden mit Hilfe des Korbs, der aus Federelementen besteht, gegen die Gefäßwand gepresst.

Des Weiteren sind Valvulotome bekannt, die im Wesentlichen aus einem Federkorb bestehen, der Schneidelemente aufweist. Ein solches in die Praxis eingeführtes Valvulotome der Firma LeMaitre ist ebenfalls kathetergeführt und weist einen Korb aus mehreren Streben mit in die Streben integrierten Schneidelementen auf. Auch diese Valvulotome sind nicht selbstständig führbar.

Weitere Valvulotome sind beispielsweise aus US 2008/255595 A1 oder US 2005/192606 A1 bekannt. Dokumente US 5,282,484 A und US 5,178,625 A offenbaren Körbe mit Schneidelementen, jedoch handelt es sich bei den Vorrichtungen um Atherektomievorrichtungen.

Demgegenüber besteht die Aufgabe, ein Valvulotom zu schaffen, das über einen Führungsdraht selbstständig an den Einsatzort führbar ist. Des Weiteren soll das Valvulotom soweit steuerbar sein, dass der Schneidvorgang vom behandelnden Arzt gesteuert und individuell an den Durchmesser und Zustand der Vene des Patienten angepasst werden kann.

Diese Aufgabe wird mit einem führungsdraht-/kathetergeführten Valvulotom mit
- einem Führungsdraht,
- einem ersten inneren Rohr zur Aufnahme des Führungsdrahts (2),
- einem expandierbaren Korb mit Schneidelementen, wobei der Korb (5) das distale Ende eines zweiten Rohres darstellt, in dem das erste innere Rohr verläuft,
- einem Katheterrohr, in dem das zweite innere Rohr verläuft, und das geeignet ist, den expandierbaren Korb in nicht expandiertem Zustand in sich aufzunehmen und zu führen, und
- einer Manschette, die das distale Ende des ersten Rohrs ausbildet und als Begrenzungs für das distale Ende des Korbs dient und durch die der Führungsdraht hindurchgeführt ist,
   gelöst.

Das erfindungsgemäße Valvulotom ist in einem herkömmlichen Katheter geführt, mit dem es auch zusammenwirkt. Es weist einen Führungsdraht auf, der über die gesamte Länge reicht und am distalen Ende des Valvulotoms mit einer gekrümmten Spitze vorsteht. Über den Führungsdraht kann das Valvulotom präzise an den Einsatzort geführt werden.

Des Weiteren verfügt das erfindungsgemäße Valvulotom über ein inneres Rohr, durch das der Führungsdraht geführt ist. Es handelt sich im Prinzip um einen Mikrokatheter, der zum einen der Führung des Führungsdrahts dient, zum anderen aber auch eine Funktion bei der Steuerung des Valvulotoms hat. Bei dem inneren Rohr kann es sich auch um ein gewickeltes Rohr mit hoher Flexibilität handeln, bei dem die Wicklungen mit einer Kunststoffhülle überzogen und/oder hinterlegt sind. Solche gewickelte Strukturen sind als Feder-Führungsdrähte mit Innenlumen bekannt.

Das erfindungsgemäße Valvulotom weist des Weiteren einen expandierbaren Korb mit Schneidelementen auf, wobei der Korb das distale Ende eines zweiten Rohrs darstellt, in dem das erste innere Rohr verläuft. Zweckmäßigerweise ist der expandierbare Korb mit den Schneidelementen einstückig gefertigt, d.h. aus einem dazu geeigneten Rohr geschnitten und geformt. Der Korb ist dann mit dem distalen Ende des zweiten Rohrs verbunden, beispielsweise durch Kleben. Das zweite Rohr kann, abgesehen von der distalen Korbstruktur, ein geeignet dimensioniertes Katheterohr sein.

Das erfindungsgemäße Valvulotom weist zudem ein Katheterrohr auf, in dem das zweite innere Rohr - und damit auch das erste innere Rohr und das Führungsrohr - verläuft und das geeignet ist, den expandierbaren Korb in nicht expandiertem Zustand in sich aufzunehmen und zu führen. Der Korb wird damit auf den Transport zu seinem Einsatzort im nicht expandierten Zustand gehalten und erst an seinem Einsatzort zur Expansion gebracht. Es ist damit ausgeschlossen, dass die Schneidelemente auf dem Transport Verletzungen erzeugen können.

Schließlich verfügt das erfindungsgemäße Valvulotom über eine Manschette, die das distale Ende des ersten Rohrs ausbildet und als Begrenzung für das distale Ende des Korbs dient und durch die der Führungsdraht - durch eine zentrale Öffnung - hindurchgeführt ist. Das distale Ende des Korbs kann gegen die Manschette beweglich angeordnet sein, die damit als Anschlag dient, oder darin festgelegt sein.

Da der Korb expandierbar ist, und damit in seinem Durchmesser variabel, erlaubt die Festlegung des distalen Ende des Korbs in der Manschette, zusammen mit dem distalen Ende des ersten Rohrs, die beiden Elemente gegeneinander zu verschieben. Mit dem ersten inneren Rohr wird das Valvulotom in Position gehalten, während das zweite Rohr mit dem distal angeordneten Korb durch Bewegung in Längsrichtung dazu verwandt werden kann, den Korb aufzuspannen oder zu strecken. Damit ist eine Anpassung des Durchmessers des Korbs an den Gefäßdurchmesser möglich und eine optimale Einstellung der Schneidelemente, insbesondere bei nicht selbstexpandierenden Korbstrukturen.

Ist das distale Ende des Korbs gegen die Manschette beweglich, gelten im Wesentlichen die gleichen Überlegungen. Der Korb kann bis zur Manschette verschoben werden, die damit als Anschlag dient, und in keinem Fall über die Manschette hinaus verschoben werden. Ein gewisser, geringer Abstand zwischen distalem Ende des Korbs und Manschette ist auch deshalb vorteilhaft, weil sich der Korb beim Zurückziehen in das Katheterrohr streckt und Platz für diese Längenexpansion benötigt. Schließlich dient die Manschette auch als Sicherheitselement, das verhindert, dass der Korb in das Gefäß des Patienten gelangt, wenn die Verbindung zwischen dem zweiten mittleren Rohr und der Korbstruktur abreißt.

Es versteht sich, dass alle diese Elemente - Führungsdraht, erstes inneres Rohr, zweites mittleres Rohr und Katheterrohr - innerhalb der gegebenen Grenzen gegeneinander beweglich sind.

Es versteht sich, dass der hier verwandte Begriff "distal" das vom behandelnden Arzt abgewandte Ende des Valvulotoms, Katheters bzw. Führungsdrahts bezeichnet.

Im Transportzustand befindet sich das erfindungsgemäße Valvulotom mit seinem Korb im Katheter, d.h. der Korb nimmt eine gestreckte Form an. Nach Freisetzung aus dem Katheter kann der Korb aufgespannt werden oder spannt sich selbst auf, je nach Material und Einstellung.

Der Korb weist selbst mehrere Streben auf, die die Schneidelemente tragen. Vorzugsweise sind es mehr als zwei Streben, insbesondere drei Streben, da sich auf diese Art und Weise das Valvulotom im Gefäß selbst zentriert.

Vorzugsweise sind die Streben distal in der Manschette zusammengeführt und dort festgelegt, beispielsweise durch Verschweißen. Dabei können die Streben individuell festgelegt sein, aber auch in einem kurzen Rohrstück zusammenlaufen, das dann in der Manschette festliegt. Neben der Möglichkeit, Manschette und Streben miteinander zu verschweißen, ist auch ein Verkrimpen oder Verkleben möglich.

Erfindungsgemäß sind die Schneidelemente am Korb angeordnet. Besteht der Korb aus einzelnen Streben, weisen die Streben diese Schneidelemente auf. Diese haben vorzugsweise nach proximal weisende hakenförmige Schneidnasen, die in der Ebene der Streben verlaufen. Insbesondere sind die Schneidnasen von Rücksprüngen der Streben flankiert, so dass sie mit ihrer Schneidfläche, insbesondere einem Schneidbogen, im Längsverlauf der Streben verbleiben. Beim Entfernen der Venenklappen werden die Venenklappen dann in dem vor der Schneide liegenden Rücksprung gehalten und durch Zurückziehen des Valvulotoms gekappt.

Vorzugsweise ist der Führungsdraht gegen das innere Rohr beweglich, so dass ein sequenzieller Vorschub des Valvulotoms möglich ist. Dazu wird der Führungsdraht jeweils vorgeschoben und der übrige Teil des Valvulotoms nachgeführt, bis der Einsatzort erreicht ist.

Zur Steuerung der Expansion des Korbs bzw. der Streben ist es insbesondere bei nicht selbstexpandierenden Korbkonstruktionen notwendig, das zweite Rohr, aus dem heraus der Korb entwickelt ist, gegen das erste innere Rohr beweglich zu halten. Dies erlaubt es dem behandelnden Arzt, durch Verschieben des zweiten Rohres gegen das erste innere Rohr den Korb aufzuspannen und hinsichtlich seiner Expansion zu kontrollieren. Nach Durchführen der Valvulotomie kann dann der Korb gestreckt werden und mit samt Manschette über das zweite Rohr in den Katheter zurückgezogen werden. Der Führungsdraht ist separat rückziehbar.

Es ist zweckmäßig, den Korb bzw. die Streben und das zweite Rohr aus einem selbstexpandierenden Material zu fertigen. Dies kann beispielsweise ein Federstahl sein, ist zweckmäßigerweise aber Nitinol, ein Material, das Formgedächtniseigenschaften aufweist. Einem Korb bzw. Streben aus Nitinol kann ein Expansionsverhalten aufgeprägt werden, so dass sich nach Freisetzung des Korbs aus dem Katheter dieser selbsttätig aufspannt, soweit es das Gefäß erlaubt. Die Flexibilität des Materials erlaubt es, den Korb nach durchgeführter Behandlung in den Katheter zurückzuziehen.

Das erfindungsgemäße Valvulotom kann übliche röntgendichte Markerelemente aufweisen, die insbesondere im Bereich der Manschette und/oder an der Katheterspitze angeordnet sein können. Beispielsweise kann auch die Manschette aus einem röntgendichten Material bestehen.

Die Erfindung wird durch die beiliegenden Abbildungen näher erläutert. Es zeigen
- Fig.1:: ein erfindungsgemäßes Valvulotom mit expandiertem Korb;
- Fig. 2:: das Valvulotom gemäß Fig. 1 mit eingefahrenem Korb;
- Fig. 3:: eine Strebe mit einem Schneidelement, und
- Fig. 4: eine weitere bevorzugte Variante des erfindungsgemäßen Valvulotoms.

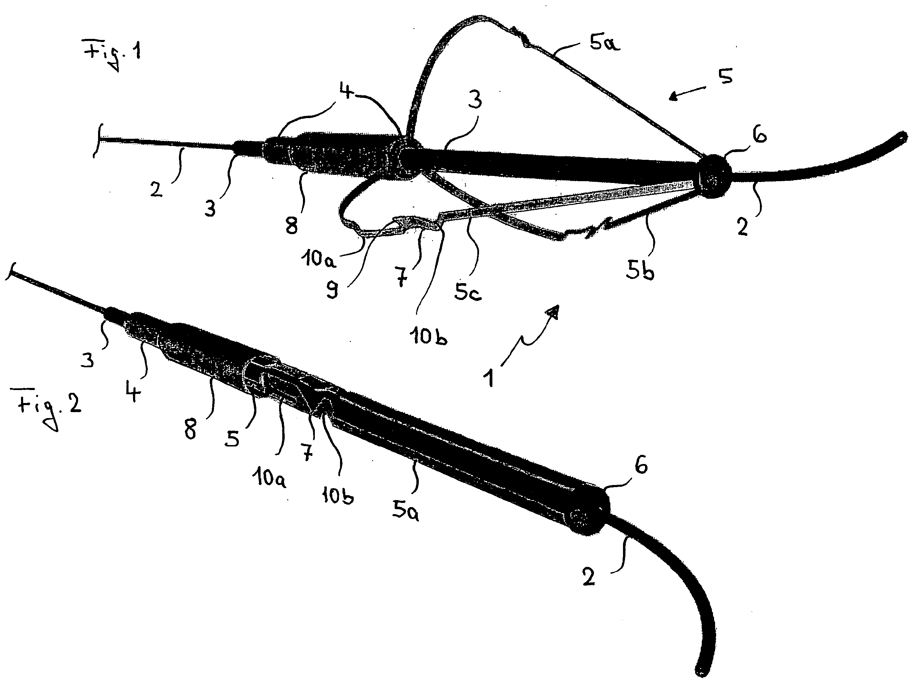

Das erfindungsgemäße Valvulotom 1 gemäß Fig. 1 weist einen zentralen Führungsdraht 2 auf, der in einem ersten inneren Rohr 3 verläuft. Der Führungsdraht 2 weist an seinem distalen Ende eine gekrümmte Spitze auf, die das Navigieren im Gefäßsystem erlaubt.

Das erste innere Rohr 3 dient der Führung des Führungsdrahts 2 und zum anderen als Steuerungselement für den Korb 5, der im gezeigten Fall aus drei Streben 5a, 5b und 5c besteht. Der Korb 5 setzt am distalen Ende eines zweiten Rohres 4 an und ist an diesem durch Verkleben befestigt. Die Streben 5a, 5b, 5c sind aus einem Rohrstück gefertigt, welches beispielsweise aus Nitinol besteht, und aus dem mit Hilfe eines Lasers die Korbstruktur 5 geschnitten ist.

Das distale Ende des ersten inneren Rohrs 3 und des zweiten Rohrs mit seinen Streben 5a, 5b, 5c sind in einer Manschette 6 festgelegt. Diese Manschette 6 stellt eine atraumatische Spitze des Valvulotoms dar, das auch bei zurückgezogenem Führungsdraht 2 von der Spitze keine Gefährdung für die Gefäßwände ausgeht. Die Manschette hat aber eine zentrale Funktion für die Beweglichkeit des Korbs 5.

In der Darstellung sind die Streben 5a, 5b, 5c des Korbs 5 aufgespannt, d.h. im expandiertem Zustand gezeigt. Dieser aufgespannte Zustand kann bei selbstexpandierenden Materialien über eine Vorspannung herbeigeführt werden, bei nicht selbstexpandierenden Materialien aber durch eine Verschiebung des zweiten Rohrs 4 in Richtung auf die Manschette bei gleichzeitigem Zurückziehen des ersten inneren Rohrs 3. Eine gegenläufige Bewegung führt in jedem Fall zur Streckung der Korbstruktur 5, die damit in den umgebenden Katheter 8 zurückgezogen werden kann.

Es versteht sich, dass der Katheter 8 wie auch die beiden Rohre 3 und 4 und der Führungsdraht jeweils über ein hämostatisches Ventil zum behandelnden Arzt geführt sind und von diesem separat betätigt werden können.

Der Korb 5 mit den Streben 5a, 5b, 5c weist die Schneidelemente 7 auf, die integraler Teil der Streben sind und nach proximal weisende hakenförmige Schneidnasen 9 aufweisen. Die Schneidelemente 7 verlaufen in der Ebene der Streben 5a, 5b, 5c. Da die Streben 5a, 5b, 5c im Wesentlichen mit ihrer Außenfläche parallel zur Gefäßwand verlaufen, schließt dies ungewollte Verletzungen der Gefäßwand durch die Klingen aus. Die Schneidnasen 9 sind vorzugsweise von Rücksprüngen 10a und 10b der Streben 5a, 5b, 5c flankiert. Dies erlaubt es, eine Venenklappe in dem proximal zur Schneidnase 9 liegenden Rücksprung 10a aufzunehmen und innerhalb dieses Rücksprungs 10a zur Schneidfläche zu führen.

Es versteht sich, dass auch das zweite Rohr 4 mit dem Korb 5 und seinen Elementen aus einem geeigneten Kunststoff gefertigt sein können, der die richtige Mischung aus Steifigkeit und Flexibilität aufweist. Die Schneidnasen können in diesem Fall mit einem schneidhaltigen Material ausgebildet sein.

Fig. 2 zeigt das Valvulotom von Fig. 1 in partiell eingefahrenem Zustand. Gleiche Bezugszeichen bezeichnen gleiche Sachverhalte. Der Korb 5 des Valvulotoms befindet sich bereits partiell im Katheter 8; die Schneidelemente 7 mit den Rücksprüngen 10a und 10b der Strebe 5a sind zu erkennen, wobei die hakenförmige Schneidnase wegen der Krümmung nicht mehr im Blickfeld ist. Zu erkennen ist, dass die Streben 5a, 5b, 5c mit Hilfe eines Schneidwerkzeugs aus einem Rohr ausgeschnitten sind.

Der gezeigte Zustand ist der Zustand, der bei der Platzierung des erfindungsgemäßen Valvulotoms unmittelbar vor dem Ausfahren des Korbs eingenommen wird bzw. nach Durchführung der Behandlung beim Einfahren des Korbs in den Katheter, bevor der Katheter zurückgezogen aus dem Körper des Patienten entfernt wird.

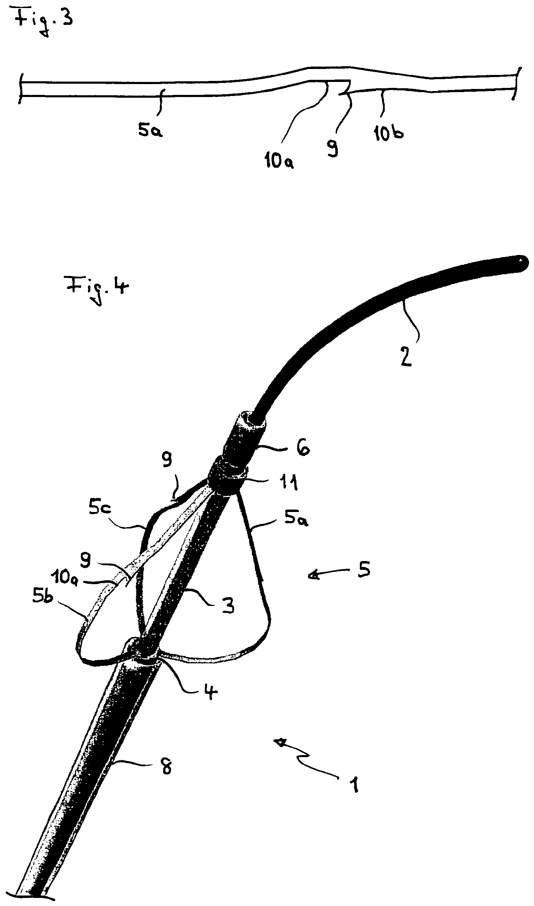

Fig. 3 zeigt eine Strebe des Korbs 5 mit der Schneidnase 9, die eine nach proximal weisende Schneide aufweist und in der Ebene der Strebe 5a angeordnet ist. Die Schneidnase 9 wird von zwei Rücksprüngen 10a und 10b der Strebe flankiert.

Fig. 4 zeigt eine bevorzugte Variante eines erfindungsgemäßen Valvulotoms, wie es schon in Fig. 1 und 2 dargestellt ist. Die Streben 5a bis 5c dieser Variante entsprechen dem in Fig. 3 gezeigten Muster. Im Übrigen zeichnen gleiche Bezugszeichen gleiche Sachverhalte.

Das Valvulotom gemäß Fig. 4 zeichnet sich durch ein gegen die Manschette 6 verschiebbares distales Ende 11 des Korbs 5 aus. Der Korb 5 setzt wie in der Ausführungsform in Fig. 1 am mittleren Rohr 4 an und führt in drei Streben 5a bis 5c zu einem rohrförmigen distalen Ende 11, das gegen die Manschette 6 geführt werden kann. Bei nicht-selbstexpandierenden Materialien für den Korb 5 führt ein Verschieben des Korbs 5 gegen die Manschette 6 dazu, dass sich die Streben 5a bis 5c unter dem Druck aufspannen. Bei einem Korb 5 aus einem selbstexpandierenden Material, etwa Nitinol, wird beim Einziehen des Korbs 5 dessen Länge vergrößert, was einen gewissen Abstand zwischen dem distalen Ende 11 und der Manschette 6 erfordert, um diese Längenvergrößerung aufzunehmen.

Im Übrigen weisen die Streben 5a bis 5c die in Fig. 3 gezeigten Schneidnasen 9 auf, die in den Rücksprüngen 10a und 10b der Streben 5a bis 5c angeordnet sind und in der Ebene der Streben 5a bis 5c liegen. In dieser Anordnung fluchten die Schneidnasen 9 im Wesentlichen mit dem Verlauf der Streben 5a bis 5c, was verhindert, dass sich die Schneidnasen beim Einziehen des Korbs 5 in das Katheterrohr 8 mit dem distalen Ende des Katheterrohrs 8 verhaken.

## Patentansprüche

1. Führungsdraht-/kathetergeführtes Valvulotom (1) mit
- einem Führungsdraht (2),
- einem ersten inneren Rohr (3) zur Aufnahme des Führungsdrahts (2),
- einem expandierbaren Korb (5) mit Schneidelementen (7), wobei der Korb (5) das distale Ende eines zweiten Rohres (4) darstellt, in dem das erste innere Rohr (3) verläuft,
- einem Katheterrohr (8), in dem das zweite innere Rohr (4) verläuft, und das geeignet ist, den expandierbaren Korb (5) in nicht expandiertem Zustand in sich aufzunehmen und zu führen,
- einer Manschette (6), die das distale Ende des ersten Rohrs (3) ausbildet und als Begrenzung für das distale Ende des Korbs (5) dient und durch die der Führungsdraht (2) hindurchgeführt ist.

2. Valvulotom nach Anspruch 1, **dadurch gekennzeichnet, dass** der Korb (5) mehrere Streben (5a, 5b, 5c) aufweist, die Schneidelemente (7) tragen.

3. Valvulotom nach Anspruch 2, **dadurch gekennzeichnet, dass** der Korb (5) drei Streben (5a, 5b, 5c) aufweist.

4. Valvulotom nach Anspruch 2 oder 3, **dadurch gekennzeichnet, dass** die Streben (5a, 5b, 5c) distal in der Manschette (6) zusammengeführt sind.

5. Valvulotom nach Anspruch 2, 3 oder 4, **dadurch gekennzeichnet, dass** die Schneidelemente (7) nach proximal weisende hakenförmige Schneidnasen (9) aufweisen, die in der Ebene der Streben (5a, 5b, 5c) verlaufen.

6. Valvulotom nach Anspruch 5, **dadurch gekennzeichnet, dass** die Schneidnasen (9) von Rücksprüngen (10a, 10b) der Streben (5a, 5b, 5c) flankiert sind.

7. Valvulotom nach einem der vorstehenden Ansprüche, **dadurch gekennzeichnet, dass** der Führungsdraht (2) gegen das innere Rohr (3) beweglich geführt ist.

8. Valvulotom nach einem der vorstehenden Ansprüche, **dadurch gekennzeichnet, dass** das zweite Rohr (4) gegen das erste innere Rohr (3) beweglich geführt ist.

9. Valvulotom nach einem der vorstehenden Ansprüche, **dadurch gekennzeichnet, dass** die Manschette (6) als atraumatische Spitze ausgebildet ist.

10. Valvulotom nach einem der vorstehenden Ansprüche, **dadurch gekennzeichnet, dass** der Korb (5) aus einem selbstexpandierenden Material besteht.

11. Valvulotom nach Anspruch 9, **dadurch gekennzeichnet, dass** das selbstexpandierende Material Nitinol ist.

12. Valvulotom nach einem der vorstehenden Ansprüche, **gekennzeichnet durch** röntgendichte Markerelemente im Bereich der Manschette (6) und/oder der Spitze des Katheters (8).

13. Valvulotom nach Anspruch 12, **dadurch gekennzeichnet, dass** die Manschette (6) aus röntgendichtem Material besteht.

## Claims

1. Valvulotome (1) guided by a guide wire/catheter, comprising
- a guide wire (2),
- a first inner tube (3) for receiving the guide wire (2),
- an expandable basket (5) having cutting elements (7), wherein the basket (5) constitutes the distal end of a second tube (4), in which the first inner tube (3) extends,
- a catheter tube (8), in which the second inner tube (4) extends and which is suitable for receiving and guiding the expandable basket (5) in the unexpanded state per se,
- a cuff (6), which forms the distal end of the first tube (3) and serves as a limit stop for the distal end of the basket (5) and through which the guide wire (2) is guided.

2. Valvulotome according to claim 1, **characterised in that** the basket (5) comprises a plurality of struts (5a, 5b, 5c) which support cutting elements (7).

3. Valvulotome according to claim 2, **characterised in that** the basket (5) comprises three struts (5a, 5b, 5c).

4. Valvulotome according to claim 2 or 3, **characterised in that** the struts (5a, 5b, 5c) are brought together distally in the cuff (6).

5. Valvulotome according to claim 2, 3 or 4, **characterised in that** the cutting elements (7) comprise hook-shaped cutting barbs (9) which point in the proximal direction and extend in the plane of the struts (5a, 5b, 5c).

6. Valvulotome according to claim 5, **characterised in that** the cutting barbs (9) are flanked by recessed portions (10a, 10b) in the struts (5a, 5b, 5c).

7. Valvulotome according to any of the preceding claims, **characterised in that** the guide wire (2) is movably guided against the inner tube (3).

8. Valvulotome according to any of the preceding claims, **characterised in that** the second tube (4) is movably guided against the first inner tube (3).

9. Valvulotome according to any of the preceding claims, **characterised in that** the cuff (6) is designed as an atraumatic tip.

10. Valvulotome according to any of the preceding claims, **characterised in that** the basket (5) consists of a self-expanding material.

11. Valvulotome according to claim 9, **characterised in that** the self-expanding material is nitinol.

12. Valvulotome according to any of the preceding claims, **characterised by** radiopaque marker elements in the region of the cuff (6) and/or the tip of the catheter (8).

13. Valvulotome according to claim 12, **characterised in that** the cuff (6) consists of radiopaque material.

## Revendications

1. Valvulotome (1) guidé par un câble de guidage et/ou un cathéter, avec
- un câble de guidage (2),
- un premier tube intérieur (3) pour recevoir le câble de guidage (2)
- un panier (5) expansible avec des éléments de coupe (7), dans lequel le panier (5) constitue l'extrémité distale d'un deuxième tube (4), dans lequel le premier tube intérieur (3) s'étend,
- un tube de cathéter (8), dans lequel le deuxième tube intérieur (4) s'étend et qui est adapté pour recevoir en son sein le panier (5) expansible dans l'état non expansé et le guider,
- un manchon (6), qui réalise l'extrémité distale du premier tube (3) et sert de délimitation pour l'extrémité distale du panier (5) et à travers lequel le câble de guidage (2) est guidé de part en part.

2. Valvulotome selon la revendication 1, **caractérisé en ce que** le panier (5) présente plusieurs entretoises (5a, 5b, 5c), qui supportent des éléments de coupe (7).

3. Valvulotome selon la revendication 2, **caractérisé en ce que** le panier (5) présente trois entretoises (5a, 5b, 5c).

4. Valvulotome selon la revendication 2 ou 3, **caractérisé en ce que** les entretoises (5a, 5b, 5c) sont regroupées distalement dans le manchon (6).

5. Valvulotome selon la revendication 2, 3 ou 4, **caractérisé en ce que** les éléments de coupe (7) présentent des ergots de coupe (9) en forme de crochets pointant vers le côté proximal, qui s'étendent dans le plan des entretoises (5a, 5b, 5c).

6. Valvulotome selon la revendication 5, **caractérisé en ce que** les ergots de coupe (9) sont flanqués de retraits (10a, 10b) des entretoises (5a, 5b, 5c).

7. Valvulotome selon l'une quelconque des revendications précédentes, **caractérisé en ce que** le câble de guidage (2) est guidé de manière mobile contre le tube intérieur (3).

8. Valvulotome selon l'une quelconque des revendications précédentes, **caractérisé en ce que** le deuxième tube (4) est guidé de manière mobile contre le premier tube intérieur (3).

9. Valvulotome selon l'une quelconque des revendications précédentes, **caractérisé en ce que** le manchon (6) est réalisé en tant que pointe atraumatique.

10. Valvulotome selon l'une quelconque des revendications précédentes, **caractérisé en ce que** le panier (5) est constitué d'un matériau auto-expansible.

11. Valvulotome selon la revendication 9, **caractérisé en ce que** le matériau auto-expansible est du Nitinol.

12. Valvulotome selon l'une quelconque des revendications précédentes, **caractérisé par** des éléments marqueurs opaques aux rayons X dans la zone du manchon (6) et/ou de la pointe du cathéter (8).

13. Valvulotome selon la revendication 12, **caractérisé en ce que** le manchon (6) est constitué d'un matériau opaque aux rayons X.
